(19) Europäisches Patentamt
European Patent Office
Office européen des brevets

(11) Veröffentlichungsnummer: **0 146 047 B1**

# (12) EUROPÄISCHE PATENTSCHRIFT

(45) Veröffentlichungstag der Patentschrift: **22.05.91**

(51) Int. Cl.5: **A01N 43/653**, A01N 43/50, A01N 43/56

(21) Anmeldenummer: **84114515.4**

(22) Anmeldetag: **30.11.84**

## (54) Mittel zur Beeinflussung des Pflanzenwachstums.

(30) Priorität: **01.12.83 DE 3343415**

(43) Veröffentlichungstag der Anmeldung:
**26.06.85 Patentblatt 85/26**

(45) Bekanntmachung des Hinweises auf die Patenterteilung:
**22.05.91 Patentblatt 91/21**

(84) Benannte Vertragsstaaten:
**BE CH DE FR GB IT LI NL**

(56) Entgegenhaltungen:
**EP-A- 0 009 740**
**EP-A- 0 113 839**
**EP-A- 0 119 572**
**DE-A- 2 657 578**

(73) Patentinhaber: **BASF Aktiengesellschaft**
**Carl-Bosch-Strasse 38**
**W-6700 Ludwigshafen(DE)**

(72) Erfinder: **Schulz, Guenter, Dr.**
**Carl-Bosch-Strasse 96**
**W-6700 Ludwigshafen(DE)**
Erfinder: **Buschmann, Ernst, Dr.**
**Georg-Ludwig-Krebs-Strasse 10**
**W-6700 Ludwigshafen(DE)**
Erfinder: **Sauter, Hubert, Dr.**
**Neckarpromenade**
**W-6800 Mannheim 1(DE)**
Erfinder: **Zeeh, Bernd, Dr.**
**Queichstrasse 5**
**W-6703 Limburgerhof(DE)**
Erfinder: **Wuerzer, Bruno, Dr. Dipl.-Landwirt**
**Ruedigerstrasse 13**
**W-6701 Otterstadt(DE)**
Erfinder: **Jung, Johann, Prof. Dr. Dipl.-Landwirt**
**Hardenburgstrasse 19**
**W-6703 Limburgerhof(DE)**
Erfinder: **Retzlaff, Guenter, Dr.**
**Schillerstrasse 34**
**W-6725 Roemerberg(DE)**

**EP 0 146 047 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft die Verwendung von Azolylacetophenonoximethern zur Bekämpfung unerwünschter Pflanzen.

Es ist bekannt, daß Azolylacetophenonoxime als Wachstumsregulator verwendet werden können (EP-A-9740). Eine Wirksamkeit solcher Oximether als Herbizide ist bisher nicht bekannt.

Es wurde nun gefunden, daß Azolylacetophenonoximether der Formel (I)

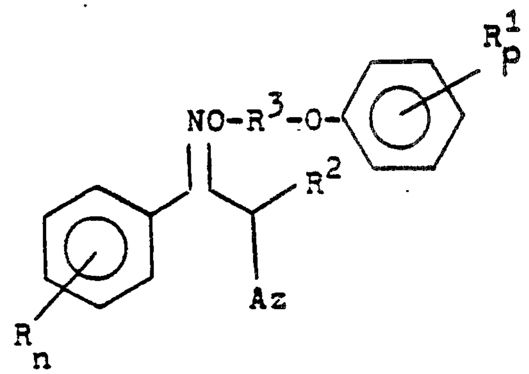

(I),

in welcher R und $R^1$ unabhängig voneinander Fluor, Chlor, Brom, $C_1$-$C_5$-Alkyl, Methoxy, Ethoxy, Methylthio, Ethylthio, $C_1$-$C_3$-Halogenalkyl mit bis zu 5 Fluor oder Chlor, Nitro, Cyano, oder ein-, zwei- oder dreifach durch Halogen, Cyano, Nitro, oder $C_1$-$C_2$-Halogenalkyl substituiertes Phenyl/Phenoxy bedeuten,
$R^2$ = Wasserstoff,
$R^3$ = $-(CH_2)_m-$, $-(CH_2CH_2O)_u-CH_2CH_2-$, $-CH_2-CH=CH-CH_2-$, $-CH_2-C{\equiv}C-CH_2-$;
n, p, u = 1, 2, 3;
m = 2 bis 8;
Az = 1-(1,2,4-Triazolyl), 1-Pyrazolyl,
oder deren Salze in Herbiziden, d.h. Mittel zur Vernichtung unerwünschter Pflanzen - im weitesten Sinne - erfolgreich verwendet werden können.

In Formel (I) bedeuten beispielsweise:
R und $R^1$ unabhängig voneinander Fluor, Chlor oder Brom, Nitro, Cyano, Alkyl mit 1 bis 4 Kohlenstoffatomen, Alkoxy- oder Alkylthio mit jeweils 1 bis 2 Kohlenstoffatomen, Halogenalkyl mit 1 bis 4 Kohlenstoffatomen und 1 bis 5 Halogenatomen, insbesondere mit 1 bis 2 Kohlenstoffatomen und 1 bis 3 gleichen oder verschiedenen Halogenatomen, wobei als Halogenatome vorzugsweise Fluor oder Chlor in Betracht kommen. Besonders bevorzugt für Halogenalkyl ist Trifluormethyl.

R und $R^1$ bedeuten außerdem unabhängig voneinander unsubstituiertes oder vorzugsweise einfach oder mehrfach (zwei- bis dreifach, gleichartig oder verschieden substituiertes Phenyl bzw. Phenoxy, wobei jeweils als Substituenten vorzugsweise in Frage kommen:
Halogen, insbesondere Fluor, Chlor oder Brom; Cyano, Nitro sowie Halogenalkyl mit 1 bis 2 Kohlenstoffatomen und bis zu 3 gleichen oder verschiedenen Halogenatomen, wobei als Halogenatome vorzugsweise Fluor oder Chlor in Betracht kommen und als Beispiel für Halogenalkyl Trifluormethyl genannt wird.

$R^2$ in Formel (I) steht für Wasserstoff;
$R^3$ in Formel (I) steht für ein Diradikal der Formel $-(CH_2)_m-$, wobei als Beispiele $-CH_2CH_2-$, $-(CH_2)_3-$, $-(CH_2)_4-$, $-(CH_2)_5-$, $-(CH_2)_6-$, $-(CH_2)_7-$ und $-(CH_2)_8-$ genannt werden.

$R^3$ kann auch ein Diradikal mit einem, zwei oder drei Sauerstoffatomen der Formel $-(CH_2CH_2O)_u-CH_2CH_2-$, wobei als Beispiele $-CH_2CH_2OCH_2CH_2-$, $-CH_2CH_2OCH_2CH_2OCH_2CH_2-$ und $-(CH_2CH_2O)_3CH_2CH_2-$ genannt werden.

Schließlich kann $R^3$ ein Diradikal mit einer Doppel- oder einer Dreifachbindung der Formel $-CH_2-CH=CH-CH_2-$ oder der Formel $-CH_2-C{\equiv}C-CH_2-$ sein.

m steht für eine ganze Zahl von 2 bis 8.

Az = 1-(1,2,4-Triazolyl) oder 1-Pyrazolyl.

Herbizide Mittel werden insbesondere dann erhalten, wenn $R^3$ das Diradikal $-(CH_2)_m-$ bedeutet, wobei m 2, 3 oder 4 ist.

Als Salze der Azolylacetophenonoximether der Formel (I) seien beispielsweise die Hydrochloride, Hydrobromide, Sulfate, Phosphate und p-Toluolsulfonate genannt.

Ein Metallkomplex ist beispielsweise das Addukt von einem Äquivalent eines Zink- oder Kupfersalzes, z. B. $CuCl_2$ oder $ZnCl_2$ an zwei Äquivalente der Verbindung (I).

Herstellungsbeispiel

2,4-Dichlor-ω-[1,2,4-triazol-1-yl-]-acetophenon-oxim-O-[2-(4-chlorphenoxy)-ethyl]ether, E- und Z-Isomeres

23 g (0,085 mol) 2,4-Dichlor-ω-[1,2,4-triazolyl-(1)]-acetophenonoxim und 20 g 2-[4-Chlorphenoxy]-bromethan werden in 100 ml Toluol mit 7,1 g gepulverter KOH und 5,8 g Triethylbenzylammoniumchlorid bei 80 bis 100°C 1 h lang kräftig gerührt. Man dampft ein, nimmt in Dichlormethan (250 ml) auf, wäscht dreimal mit je 100 ml Wasser, trocknet die organische Phase mit Natriumsulfat und dampft ein. Chromatographie an Kieselgel mit Dichlormethan als Elutionsmittel liefert 6 g des Z-Isomeren (Wirkstoff Nr. 23) und 4 g des E-Isomeren des 2,4-Dichlor-ω-[1,2,4-triazol-1-yl]-acetophenon-oxim-O-[2-(4-chlorphenoxy)-ethyl]ethers (Wirkstoff Nr. 24). Das Z-Isomere wird zuerst eluiert.

Entsprechend können die in der folgenden Tabelle aufgeführten Verbindungen hergestellt werden. Dabei wird die Konfiguration, bei der der Oximsauerstoff und der C-Aromat auf der gleichen Seite der Oximdoppelbindung stehen mit E bezeichnet, während das entsprechende andere Isomere die Bezeichnung Z erhält.

Tri = 1-(1,2,4-Triazolyl),Pyr = 1-Pyrazolyl, Im = Imidazolyl, Ts = Tosyl, Bu = Butyl, Me = Methyl.

Entsprechend wurden diejenigen in der folgenden Tabelle angegebenen Verbindungen der Formel I hergestellt, deren Schmelzpunkte (Fp) oder charakteristische Banden angegeben sind. Ihre Struktur wurde durch Elementaranalyse gesichert. Die Verbindungen, bei denen keine Angaben zur physikalischen Chemie gemacht sind, können auf die gleiche Art und Weise, wie bei den hergestellten Verbindungen beschrieben, erhalten werden; es kann erwartet werden, daß sie aufgrund ihrer ähnlichen Konstitution ähnliche Wirkungen wie die näher untersuchten Verbindungen haben.

**Tabelle A**

Verbindungen der Formel I mit $R^3 = -(CH_2)_m-$:

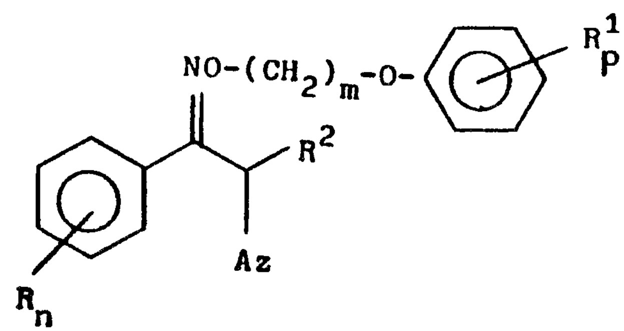

| Nr. | $R_n$ | $R^1_p$ | $R^2$ | Az | Konfiguration | m | Fp [°C] |
|---|---|---|---|---|---|---|---|

<u>Tabelle A</u> (Fortsetzung)

| Nr. | $R_n$ | $R^1_p$ | $R^2$ | Az | Konfiguration | m | Fp [°C] |
|---|---|---|---|---|---|---|---|
| 23 | 2,4-$Cl_2$ | 4-Cl | H | Tri | Z | 2 | 87 - 91 |
| 24 | 2,4-$Cl_2$ | 4-Cl | H | Tri | E | 2 | 58 - 61 |
| 25 | 2,4-$Cl_2$ | 4-Cl | H | Tri.$HNO_3$ | E/Z-Gemisch | 2 | |
| 26 | 2,4-$Cl_2$ | 4-Cl | H | Tri.$HNO_3$ | E/Z-Gemisch | 2 | |
| 27 | 2,4-$Cl_2$ | 4-Cl | H | Tri.$H_2SO_4$ | E/Z-Gemisch | 2 | |
| 28 | 2,4-$Cl_2$ | 4-Cl | H | Tri.$H_3PO_4$ | E/Z-Gemisch | 2 | |
| 29 | 2,4-$Cl_2$ | 4-Cl | H | Tri.TsOH | E/Z-Gemisch | 2 | |
| 30 | 2,4-$Cl_2$ | 4-Cl | H | Tri. 1/2 $CuCl_2$ | E/Z-Gemisch | 2 | |
| 31 | 2,4-$Cl_2$ | 4-Cl | H | Tri. 1/2 $ZnCl_2$ | E/Z-Gemisch | 2 | |
| 36 | 2,4-$Cl_2$ | 4-Cl | H | Tri | Z | 3 | Öl |

Tabelle A (Fortsetzung)

| Nr. | $R_n$ | $R^1_p$ | $R^2$ | Az | Konfiguration | m | Fp [°C] |
|---|---|---|---|---|---|---|---|
| 37 | 2,4-$Cl_2$ | 4-Cl | H | Tri | E | 3 | Öl |
| 38 | 2,4-$Cl_2$ | 4-Cl | H | Tri | Z | 4 | 65 - 71 |
| 39 | 2,4-$Cl_2$ | 4-Cl | H | Tri | E | 4 | Öl |
| 40 | 2,4-$Cl_2$ | 4-Cl | H | Tri | Z | 5 | Öl |
| 41 | 2,4-$Cl_2$ | 4-Cl | H | Tri | E | 5 | 59 - 62 |
| 42 | 2,4-$Cl_2$ | 2,4-$Cl_2$ | H | Tri | Z | 2 | 95 - 100 |
| 43 | 2,4-$Cl_2$ | 2,4-$Cl_2$ | H | Tri | E | 2 | Öl |
| 48 | 2,4-$Cl_2$ | 2,4-$Cl_2$ | H | Tri.$HNO_3$ | E/Z-Gemisch | 2 | 132 (Zers.) |
| 49 | 2,4-$Cl_2$ | 2,4,6-$Cl_3$ | H | Tri.HCl | E/Z-Gemisch | 2 | |
| 50 | 2,4-$Cl_2$ | 2,4,6-$Cl_3$ | H | Tri. 1/2 $ZnCl_2$ | E/Z-Gemisch | 2 | |
| 51 | 2,4-$Cl_2$ | 2,4,6-$Cl_3$ | H | Tri. 1/2 $CuCl_2$ | E/Z-Gemisch | 2 | |
| 52 | 2,4-$Cl_2$ | 4-F | H | Tri | Z | 2 | 66 - 70 |
| 53 | 2,4-$Cl_2$ | 4-F | H | Tri | E | 2 | 73 - 77 |
| 54 | 2,4-$Cl_2$ | 4-CN | H | Tri | Z | 2 | 96 - 100 |
| 55 | 2,4-$Cl_2$ | 4-CN | H | Tri | E | 2 | |
| 56 | 2,4-$Cl_2$ | 4-tert.-Bu | H | Tri.$HNO_3$ | E/Z-Gemisch | 2 | 117 |
| 57 | 2,4-$Cl_2$ | 4-OMe | H | Tri | Z | 2 | Öl |
| 58 | 2,4-$Cl_2$ | 4-OMe | H | Tri | E | 2 | |
| 59 | 2,4-$Cl_2$ | 4-SMe | H | Tri | E/Z-Gemisch | 2 | |
| 60 | 2,4-$Cl_2$ | 4-Br | H | Tri | E/Z-Gemisch | 2 | |

Tabelle A (Fortsetzung)

| Nr. | $R_n$ | $R^1_p$ | $R^2$ | Az | Konfiguration | m | Fp [°C] |
|---|---|---|---|---|---|---|---|
| 61 | 2,4-$Cl_2$ | 4-$NO_2$ | H | Tri | E/Z-Gemisch | 2 | |
| 62 | 2,4-$Cl_2$ | 3-Cl | H | Tri | Z | 2 | Öl |
| 63 | 2,4-$Cl_2$ | 3-Cl | H | Tri | E | 2 | 73 - 77 |
| 64 | 2,4-$Cl_2$ | 3-$CF_3$ | H | Tri | Z | 2 | Öl |
| 65 | 2,4-$Cl_2$ | 3-$CF_3$ | H | Tri | E | 2 | 66 - 69 |
| 69 | 2,4-$Cl_2$-3-$CH_3$ | 4-Cl | H | Tri | E/Z-Gemisch | 2 | Öl |
| 70 | 2,5-$Cl_2$ | 4-Cl | H | Tri | Z | 2 | Öl |
| 76 | 2,4-$Cl_2$ | 4-Cl | H | Pyr | E/Z-Gemisch | 2 | |
| 77 | 2,4-$Cl_2$ | 4-F | H | Pyr | E/Z-Gemisch | 2 | |
| 78 | 2,4-$Cl_2$ | 4-CN | H | Pyr | E/Z-Gemisch | 2 | |
| 79 | 2,4-$Cl_2$ | 4-OMe | H | Pyr | E/Z-Gemisch | 2 | |
| 114 | 2,4-$Cl_2$ | 2,4,6-$Cl_3$ | H | Tri | Z-Isomer | 2 | 76 - 85 |
| 127 | 4-Cl | 2,4,6-$Cl_3$ | H | Tri | Z-Isomer | 2 | 114 - 116 |

**Tabelle A (Fortsetzung)**

| Nr. | $R_n$ | $R^1_p$ | $R^2$ | Az | Konfiguration | m | Fp [°C] |
|---|---|---|---|---|---|---|---|
| 148 | $4-CH_3$ | 4-Cl | H | Tri | Z-Isomer | 2 | 110 - 112 |
| 149 | $4-CH_3$ | 4-Cl | H | Tri · HCl | Z-Isomer | 2 | 164 - 167 |
| 150 | $4-CH_3$ | 3-Cl | H | Tri | Z-Isomer | 2 | 86 - 91 |
| 151 | $4-CH_3$ | 3-Cl | H | Tri · HCl | Z-Isomer | 2 | Öl |
| 152 | $4-CH_3$ | $3-CH_3$ | H | Tri | Z-Isomer | 2 | 66 - 71 |
| 153 | $4-CH_3$ | $3-CH_3$ | H | Tri · HCl | Z-Isomer | 2 | 125 - 128 |

Tabelle A (Fortsetzung)

| Nr. | $R_n$ | $R^1_p$ | $R^2$ | Az | Konfiguration | m | Fp [°C] |
|---|---|---|---|---|---|---|---|
| 154 | 4-$CH_3$ | 4-Cl | H | Tri | Z-Isomer | 3 | 70 - 72 |
| 155 | 4-$CH_3$ | 4-Cl | H | Tri • HCl | Z-Isomer | 3 | 160 - 163 |
| 158 | 4-$CH_3$ | 4-Cl | H | Tri | Z-Isomer | 4 | 75 - 76 |
| 159 | 4-$CH_3$ | 4-Cl | H | Tri • HCl | Z-Isomer | 4 | 123 - 125 |
| 160 | 4-Cl | 4-Cl | H | Tri | Z-Isomer | 2 | 133 - 135 |
| 161 | 4-Cl | 4-Cl | H | Tri • HCl | Z-Isomer | 2 | 174 - 178 |
| 162 | 4-Cl | 4-Cl | H | Tri | Z-Isomer | 4 | 75 - 81 |
| 163 | 4-Cl | 4-Cl | H | Tri • HCl | Z-Isomer | 4 | 128 - 130 |
| 166 | 4-Cl | 3-Cl | H | Tri | Z-Isomer | 2 | 89 - 94 |
| 167 | 4-Cl | 3-Cl | H | Tri • HCl | Z-Isomer | 2 | 141 - 145 |
| 168 | 4-Cl | 3-$CH_3$ | H | Tri | Z-Isomer | 2 | 103 - 105 |
| 169 | 4-Cl | 3-$CH_3$ | H | Tri • HCl | Z-Isomer | 2 | 107 - 108 |
| 179 | 4-Cl | 3-$CF_3$ | H | Tri | | 2 | 89 - 93 |
| 180 | 4-Cl | 3,5-$Cl_2$ | H | Tri | | 2 | 88 - 94 |
| 181 | 4-$CH_3$ | 3,5-$Cl_2$ | H | Tri | | 2 | 70 - 74 |
| 182 | 4-Cl | 3-Cl | H | Tri | | 3 | 67 - 69 |
| 183 | 4-$CH_3$ | 3-Cl | H | Tri | | 2 | 54 - 57 |

Tabelle A (Fortsetzung)

| Nr. | $R_n$ | $R_p^1$ | $R^2$ | Az | Konfiguration | m | Fp [°C] |
|---|---|---|---|---|---|---|---|
| 185 | 4-Cl | 3-Cl | H | Pyr | | 2 | 73 - 78 |
| 188 | 4-Cl | 3,4-$Cl_2$ | H | Tri | Z-Isomer | 2 | 83 |
| 189 | 4-Cl | 2,3-$Cl_2$ | H | Tri | Z-Isomer | 2 | 103 - 106 |
| 190 | 4-Cl | 3-$OCH_3$ | H | Tri | Z-Isomer | 2 | 99 |
| 191 | 3,4-$Cl_2$ | 3-Cl | H | Tri | Z-Isomer | 2 | 84 |
| 192 | 4-F | 3-Cl | H | Tri | Z-Isomer | 2 | 71 - 74 |
| 193 | 3,4-$Cl_2$ | 3-$CH_3$ | H | Tri | Z-Isomer | 2 | 73 - 75 |
| 194 | 4-F | 3-$CH_3$ | H | Tri | Z-Isomer | 2 | 63 - 65 |
| 195 | 4-Cl | 3-$NO_2$ | H | Tri | Z-Isomer | 2 | 135 - 137 |
| 198 | 4-Cl | 2,4,5-$Cl_3$ | H | Tri | Z/E-Gemisch | 2 | 89 |
| 199 | 4-Cl | 2,5-$Cl_2$ | H | Tri | Z/E-Gemisch | 2 | 124 - 126 |
| 200 | 4-$OCH_3$ | 3-$CF_3$ | H | Tri | Z/E-Gemisch | 2 | 64 - 67 |
| 202 | 4-F | 3-$CF_3$ | H | Tri | Z/E-Gemisch | 2 | 70 - 74 |
| 203 | 3,4-$Cl_2$ | 3-$CF_3$ | H | Tri | Z/E-Gemisch | 2 | 90 - 94 |
| 204 | 4-$OCH_3$ | 3-Cl | H | Tri | Z/E-Gemisch | 2 | 48 - 50 |

Tabelle A (Fortsetzung)

| Nr. | $R_n$ | $R^1_p$ | $R^2$ | Az | Konfiguration | m | Fp [°C] |
|---|---|---|---|---|---|---|---|
| 205 | 4-$OCH_3$ | 3-$CH_3$ | H | Tri | Z/E-Gemisch | 2 | Öl |
| 207 | 4-$(CH_3)_2CH$- | 3-Cl | H | Tri | Z/E-Gemisch | 2 | |
| 208 | 4-$(CH_3)_2CH$- | 3-$CH_3$ | H | Tri | Z/E-Gemisch | 2 | |
| 209 | 4-$(CH_3)_2CH$- | 3,4-$Cl_2$ | H | Tri | Z/E-Gemisch | 2 | |
| 210 | 4-$(CH_3)_2CH$- | 2,3-$Cl_2$ | H | Tri | Z/E-Gemisch | 2 | |
| 211 | 4-$(CH_3)_2C$- | 3-$CH_3$ | H | Tri | Z/E-Gemisch | 2 | |
| 212 | 4-$(CH_3)_2C$- | 3-Cl | H | Tri | Z/E-Gemisch | 2 | |
| 213 | 4-$(CH_3)_3C$- | 3,4-$Cl_2$ | H | Tri | Z/E-Gemisch | 2 | |
| 214 | 4-$(CH)_3C$- | 2,3-$Cl_2$ | H | Tri | Z/E-Gemisch | 2 | |
| 221 | 4-$OCH_3$ | 2,3-$Cl_2$ | H | Tri | Z/E-Gemisch | 2 | |
| 222 | 4-$OCH_3$ | 3,4-$Cl_2$ | H | Tri | Z/E-Gemisch | 2 | |

Tabelle A (Fortsetzung)

| Nr. | $R_n$ | $R^1_p$ | $R^2$ | Az | Konfiguration | m |
|---|---|---|---|---|---|---|
| 237 | 4-$NO_2$ | 3-Cl | H | Tri | Z/E-Gemisch | 2 |
| 238 | 4-$NO_2$ | 3-$CH_3$ | H | Tri | Z/E-Gemisch | 2 |
| 239 | 4-$NO_2$ | 3,4-$Cl_2$ | H | Tri | Z/E-Gemisch | 2 |
| 240 | 4-$NO_2$ | 2,3-$Cl_2$ | H | Tri | Z/E-Gemisch | 2 |
| 241 | 2-Cl | 3-Cl | H | Tri | Z/E-Gemisch | 2 |
| 242 | 2-Cl | 3-$CH_3$ | H | Tri | Z/E-Gemisch | 2 |
| 243 | 2-Cl | 2,3-$Cl_2$ | H | Tri | Z/E-Gemisch | 2 |
| 244 | 2-Cl | 3,4-$Cl_2$ | H | Tri | Z/E-Gemisch | 2 |
| 245 | 3,5-$Cl_2$ | 3-Cl | H | Tri | Z/E-Gemisch | 2 |
| 246 | 3,5-$Cl_2$ | 3-$CH_3$ | H | Tri | Z/E-Gemisch | 2 |
| 247 | 4-Br | 3-Cl | H | Tri | Z/E-Gemisch | 2 |
| 255 | 4-F | 3-F | H | Tri | Z/E-Gemisch | 2 |

Tabelle A (Fortsetzung)

| Nr. | $R_n$ | $R^1_p$ | $R^2$ | Az | Konfiguration | m | Fp [°C] |
|---|---|---|---|---|---|---|---|
| 260 | 4-$CH_3$ | 2-F | H | Tri | Z | 2 | |
| 261 | 4-$CH_3$ | 2-F | H | Tri | E | 2 | |
| 262 | 4-$CH_3$ | 2-Cl | H | Tri | Z | 2 | |
| 263 | 4-$CH_3$ | 2-Cl | H | Tri | E | 2 | |
| 272 | 4-Cl | 2-F | H | Tri | Z | 2 | |
| 273 | 4-Cl | 2-F | H | Tri | E | 2 | |
| 274 | 4-Cl | 2-Cl | H | Tri | Z | 2 | |
| 275 | 4-Cl | 2-Cl | H | Tri | E | 2 | |
| 276 | 4-F | 2-Cl | H | Tri | E/Z-Gemisch | 2 | 74 - 77 |

Tabelle A (Fortsetzung)

| Nr. | $R_n$ | $R^1_p$ | $R^2$ | Az | Konfiguration | m | Fp [°C] |
|---|---|---|---|---|---|---|---|
| 277 | 4-F | 2-Cl | H | Tri | Z | 2 | 81 |
| 278 | 4-F | 2-Cl | H | Tri | E | 2 | |
| 279 | 4-F | 2-F | H | Tri | Z | 2 | 86 |
| 280 | 4-F | 2-F | H | Tri | E | 2 | |
| 281 | 4-$OCH_3$ | 2-F | H | Tri | Z | 2 | 79 - 81 |
| 282 | 4-$OCH_3$ | 2-F | H | Tri | E | 2 | |
| 283 | 4-$OCH_3$ | 2-Cl | H | Tri | Z | 2 | 71 |
| 284 | 4-$OCH_3$ | 2-Cl | H | Tri | E | 2 | |
| 285 | 4-CN | 2-F | H | Tri | Z | 2 | |
| 286 | 4-CN | 2-F | H | Tri | E | 2 | |
| 289 | 4-$CH_3$ | 2-CN | H | Tri | Z | 2 | |
| 290 | 4-$CH_3$ | 2-CN | H | Tri | E | 2 | |
| 291 | 4-Cl | 2-CN | H | Tri | Z | 2 | |
| 292 | 4-Cl | 2-CN | H | Tri | E | 2 | |
| 293 | 4-F | 2-CN | H | Tri | Z | 2 | |
| 294 | 4-F | 2-CN | H | Tri | E | 2 | |
| 297 | 4-$CH_3$ | 2-$CH_3$ | H | Tri | Z | 2 | |

Tabelle A (Fortsetzung)

| Nr. | $R_n$ | $R^1_p$ | $R^2$ | Az | Konfiguration | m | Fp [°C] |
|---|---|---|---|---|---|---|---|
| 298 | 4-$CH_3$ | 2-$CH_3$ | H | Tri | E | 2 | |
| 299 | 4-Cl | 2-$CH_3$ | H | Tri | Z | 2 | |
| 300 | 4-Cl | 2-$CH_3$ | H | Tri | E | 2 | |
| 301 | 4-F | 2-$CH_3$ | H | Tri | Z | 2 | |
| 302 | 4-F | 2-$CH_3$ | H | Tri | E | 2 | |
| 305 | 2-$CH_3$ | 2-$OCH_3$ | H | Tri | Z | 2 | |
| 306 | 2-$CH_3$ | 2-$OCH_3$ | H | Tri | E | 2 | |
| 307 | 4-Cl | 2-$OCH_3$ | H | Tri | Z | 2 | |
| 308 | 4-Cl | 2-$OCH_3$ | H | Tri | E | 2 | |
| 309 | 4-F | 2-$OCH_3$ | H | Tri | Z | 2 | |
| 310 | 4-F | 2-$OCH_3$ | H | Tri | E | 2 | |
| 311 | 3-Cl | 2-Cl | H | Tri | Z | 2 | |
| 312 | 3-Cl | 2-F | H | Tri | Z | 2 | |
| 313 | 3-Br | 2-Cl | H | Tri | Z | 2 | |
| 314 | 3,4-$Cl_2$ | 2-Cl | H | Tri | Z | 2 | 98 |
| 315 | 3,4-$Cl_2$ | 2-F | H | Tri | Z | 2 | 103-105 |
| 316 | 3,5-$Cl_2$ | 2-F | H | Tri | Z | 2 | 117 |
| 317 | 3,5-$Cl_2$ | 2-Cl | H | Tri | Z | 2 | 108 |
| 318 | 2,5-$Cl_2$ | 2-F | H | Tri . $HNO_3$ | Z | 2 | 110 |

Tabelle A (Fortsetzung)

| Nr. | $R_n$ | $R_p^1$ | $R^2$ | Az | Konfiguration | m | Fp [°C] |
|---|---|---|---|---|---|---|---|
| 319 | 2,5-$Cl_2$ | 2-Cl | H | Tri | Z | 2 | wachsartig |
| 320 | 2,4-$Cl_2$ | 2-F | H | Tri | Z | 2 | |
| 321 | 2,4-$Cl_2$ | 2-Cl | H | Tri | Z | 2 | |
| 322 | 2-Cl | 2-F | H | Tri | Z | 2 | |
| 323 | 2-Cl | 2-Cl | H | Tri | Z | 2 | |
| 325 | 4-$CH_3$ | 2,6-$Cl_2$ | H | Tri | Z | 2 | |
| 326 | 4-Cl | 2,6-$Cl_2$ | H | Tri | Z | 2 | |
| 327 | 4-F | 2,6-$Cl_2$ | H | Tri | Z | 2 | |
| 328 | 2,5-$Cl_2$ | 3-Cl | H | Tri | Z | 2 | Öl |
| 329 | 3-Br | 3-Cl | H | Tri | Z | 2 | 100-103 |

Tabelle B

Verbindungen der Formel I mit $R^3 = -(CH_2CH_2O)_uCH_2CH_2$

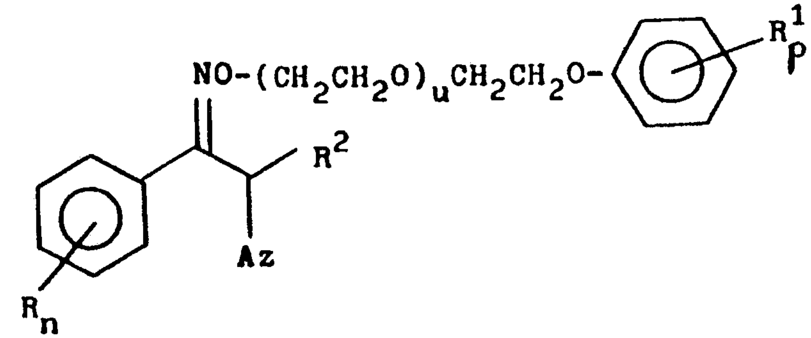

| Nr. | $R_n$ | $R^1_p$ | $R^2$ | Az | Konfiguration | u | Fp [°C] |
|---|---|---|---|---|---|---|---|
| 84 | 2,4-$Cl_2$ | 4-Cl | H | Tri | E/Z-Gemisch | 1 | |
| 85 | 2,4-$Cl_2$ | 4-Cl | H | Tri | E/Z-Gemisch | 2 | Öl |
| 86 | 2,4-$Cl_2$ | 4-Cl | H | Tri | E/Z-Gemisch | 3 | |
| 87 | 2,4-$Cl_2$ | 2,4-$Cl_2$ | H | Tri | E/Z-Gemisch | 1 | |
| 88 | 2,4-$Cl_2$ | 2,4-$Cl_2$ | H | Tri | E/Z-Gemisch | 2 | Öl |
| 89 | 2,4-$Cl_2$ | 2,4-$Cl_2$ | H | Tri | E/Z-Gemisch | 3 | |
| 106 | 2,4-$Cl_2$ | 2-Cl | H | Tri | E/Z-Gemisch | 2 | Öl |
| 107 | 2,4-$Cl_2$ | 3-Cl | H | Tri | E/Z-Gemisch | 2 | Öl |
| 108 | 2,4-$Cl_2$ | 4-$OCH_3$ | H | Tri | Z-Isomer | 2 | Öl |

Tabelle B (Fortsetzung)

| Nr. | $R_n$ | $R^1_p$ | $R^2$ | Az | Konfiguration | u | Fp [°C] |
|---|---|---|---|---|---|---|---|
| 109 | 2,4-$Cl_2$ | 2,4,6-$Cl_3$ | H | Tri | Z-Isomer | 2 | Öl |
| 110 | 2,4-$Cl_2$ | 2-Cl | H | Tri | E/Z-Gemisch | 1 | wachsartig |
| 111 | 2,4-$Cl_2$ | 4-$OCH_3$ | H | Tri | Z-Isomer | 1 | wachsartig |
| 112 | 2,4-$Cl_2$ | 3-$CH_3$ | H | Tri | Z-Isomer | 1 | wachsartig |
| 113 | 2,4-$Cl_2$ | 4-$CH_3$ | H | Tri | Z-Isomer | 1 | wachsartig |
| 116 | 4-Cl | 2,4,6-$Cl_3$ | H | Tri | Z-Isomer | 1 | 69 - 71 |
| 117 | 4-Cl | 2-Cl | H | Tri | Z-Isomer | 1 | 63 - 65 |
| 118 | 4-Cl | 3-$CH_3$ | H | Tri | Z-Isomer | 1 | 66 - 68 |
| 119 | 4-Cl | 4-$OCH_3$ | H | Tri | Z-Isomer | 1 | 71 |
| 120 | 4-Cl | 4-$CH_3$ | H | Tri | Z-Isomer | 1 | 79 |
| 122 | 4-Cl | 4-Cl | H | Tri | Z-Isomer | 2 | 85 - 88 |
| 123 | 4-Cl | 2-Cl | H | Tri | Z-Isomer | 2 | Öl |
| 124 | 4-Cl | 3-Cl | H | Tri | Z-Isomer | 2 | Öl |
| 125 | 4-Cl | 2,4-$Cl_2$ | H | Tri | Z-Isomer | 2 | 48 - 53 |
| 126 | 4-Cl | 4-$OCH_3$ | H | Tri | Z-Isomer | 2 | Öl |
| 170 | 4-$CH_3$ | 4-Cl | H | Tri | Z-Isomer | 2 | Öl |

Tabelle C

Verbindungen der Formel I mit $R^3 = -(CH_2)_v-CH=CH-(CH_2)_t-$

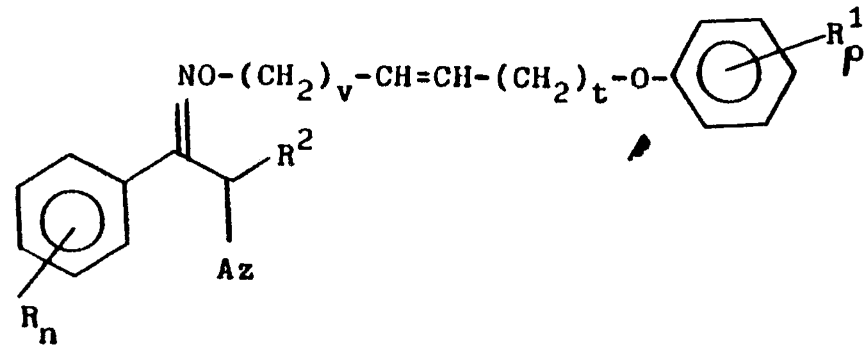

| Nr. | $R_n$ | $R^1_p$ | $R^2$ | Az | Konfiguration | v | t | Fp [°C] |
|---|---|---|---|---|---|---|---|---|
| 90 | $2,4-Cl_2$ | 4 | H | Tri | E/Z-Gemisch | 1 | 1 | Öl |
| 91 | $2,4-Cl_2$ | 4-Cl | H | Tri | E/Z-Gemisch | 1 | 1 | Öl |
| 92 | $2,4-Cl_2$ | $4-CH_3$ | H | Tri | E/Z-Gemisch | 1 | 1 | |
| 93 | $2,4-Cl_2$ | $4-OCH_3$ | H | Tri | E/Z-Gemisch | 1 | 1 | |
| 94 | $2,4-Cl_2$ | $2,4-Cl_2$ | H | Tri | E/Z-Gemisch | 1 | 1 | Öl |
| 95 | $2,4-Cl_2$ | $2,4,6-Cl_3$ | H | Tri | E/Z-Gemisch | 1 | 1 | Öl |
| 115 | $2,4-Cl_2$ | 2-Cl | H | Tri | E/Z-Gemisch | 1 | 1 | 84 |

Tabelle D

Verbindungen der Formel I mit $R^3 = -(CH_2)_v-C=C-(CH_2)_t-$

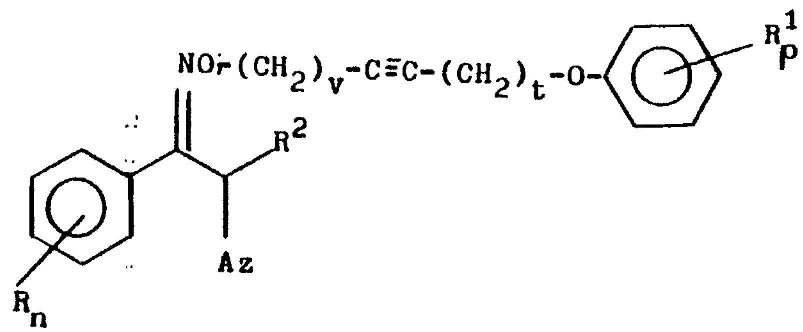

| Nr. | $R_n$ | $R^1_p$ | $R^2$ | Az | Konfiguration | v | t | Fp [°C] |
|---|---|---|---|---|---|---|---|---|
| 99 | 2,4-$Cl_2$ | 4-Cl | H | Tri | E/Z-Gemisch | 1 | 1 | Öl |
| 100 | 2,4-$Cl_2$ | 4-$CH_3$ | H | Tri | E/Z-Gemisch | 1 | 1 | Öl |
| 101 | 2,4-$Cl_2$ | 4-$OCH_3$ | H | Tri | E/Z-Gemisch | 1 | 1 | Öl |
| 102 | 2,4-$Cl_2$ | 2,4-$Cl_2$ | H | Tri | E/Z-Gemisch | 1 | 1 | Öl |
| 103 | 2,4-$Cl_2$ | 3-$CH_3$ | H | Tri | E/Z-Gemisch | 1 | 1 | Öl |
| 172 | 2,4-$Cl_2$ | 4-F | H | Tri | Z-Isomer | 1 | 1 | Öl |
| 173 | 2,4-$Cl_2$ | 3-Cl | H | Tri | Z-Isomer | 1 | 1 | Öl |
| 175 | 4-Cl | 4-Cl | H | Tri | Z-Isomer | 1 | 1 | 81 - 85 |
| 176 | 4-Cl | 4-F | H | Tri | Z-Isomer | 1 | 1 | 70 - 75 |
| 177 | 4-Cl | 3-Cl | H | Tri | Z-Isomer | 1 | 1 | 80 - 85 |
| 178 | 4-Cl | 4-$OCH_3$ | H | Tri | Z-Isomer | 1 | 1 | Öl |

Charakteristische kernresonanzspektroskopische Daten ($^1$H-NMR):

| Nr. | δ in ppm, Lsgm. $CDCl_3$ |
|---|---|
| 36 | 4,03 "t" 2H; 4,43 "t" 2H; 5,44 s 2H; 7,80 s 1H; 8,07 s 1H |
| 37 | 3,90 "t" 2H; 4,27 "t" 2H; 5,20 s 2H; 7,90 s 1H; 8,00 s 1H |
| 39 | 3,78 "t" 2H; 4,08 "t" 2H; 5,05 s 2H; 7,70 s 1H; 7,85 s 1H |
| 40 | 3,38 "t" 2H; 4,20 "t" 2H; 5,35 s 2H; 7,70 s 1H; 7,98 s 1H |
| 43 | 4,22 "t" 2H; 4,48 "t" 2H;, 5,23 s 2H; 7,98 s 1H; 8,03 s 1H |
| 57 | 4,25 "t" 2H; 4,60 "t" 2H; 5,47 s 2H;; 7,80 s 1H; 8,17 s 1H |
| 62 | 4,27 "t" 2H; 4,60 "t" 2H; 5,48 s 2H; 7,80 s 1H; 8,15 s 1H |
| 64 | 4,43 "t" 2H; 4,63 "t" 2H;, 545 s 2H; 7,80 s 1H; 8,13 s 1H |
| 69 | 2,41 3H; 4,22 "t" 2H; 4,58 "t" 2H; 5,41 s 2H; 7,78 s 1H; 8,11 s 1H |
| 70 | 4,22 "t" 2H; 4,59 "t" 2H; 5,42 s 2H; 7,81 s 1H; 8,14 s 1H |
| 100 | 2,28 s 3H; 4,70 m 3H; 5,20 s 2H; 7,90 s 1H; 8,00 s 1H |
| 101 | 3,72 s 3H; 4,70 s 2H; 4,87 s 2H; 5,44 s 2H; 7,80 s 1H; 8,11 s 1H |

Charakteristische kernresonanzspektroskopische Daten ($^1H$-NMR):

| Nr. | δ in ppm, Lsgm. $CDCl_3$ |
|---|---|
| 102 | 4,70 m 4H; 5,20 s und 5,50 s 2H; 7,80 s und 7,94 s 1H; 8,01 s und 8,10 s 1H |
| 103 | 2,31 s 3H; 4,72 s 2H; 4,87 s 2H; 5,44 s 2H; 7,80 s 1H; 8,10 s 1H |
| 112 | 3,75 m 4H; 4,15 m 2H; 4,35 m 2H, 5,53 s 2H; 7,83 s 1H; 8,50 s 1H |
| 113 | 3,75 m 4H; 4,15 m 2H; 4,38 m 2H; 5,53 s 2H; 7,85 s 1H; 8,50 s 1H |
| 123 | 3,80 m 8H; 4,15 m 2H; 4,45 m 2H; 5,38 s 2H; 7,88 s 1H; 8,38 s 1H |
| 124 | 3,70 m 8H; 4,15 m 2H; 4,30 m 2H; 5,45 s 2H; 7,78 s 1H; 8,45 s 1H |
| 126 | 3,80 m 8H; 4,10 m 2H; 4,40 m 2H; 5,60 s 2H; 8,05 s 1H; 8,70 s 1H |
| 170 | 3,70 m 8H; 4,00 m 2H; 4,35 m 2H; 5,30 s 2H; 7,75 s 1H; 8,43 s 1H |
| 172 | 4,60 "t" 2H; 4,80 "t" 2H; 5,40 s 2H;, 7,70 s 1H; 7,98 s 1H |
| 173 | 4,65 "t" 2H; 4,85 "t" 2H; 5,40 s 2H;, 7,70 s 1H; 8,00 s 1H |
| 106 | 3,7 m 8H; 4,2 m 4H; 5,55 s 2H; 7,87 s 1H; 8,52 s 1 H |
| 107 | 3,7 m 8H; 4,2 m 4H; 5,57 s 2H; 7,85 s 1H; 8,50 s 1H |
| 108 | 3,7 m 8H; 4,0 m 2H;; 4,45 m 2H; 5,55 s 2H; 7,88 s 1H; 8,5 s 1H |

Charakteristische kernresonanzspektroskopische Daten ($^1H$-NMR):

| Nr. | in ppm, Lsgm. $CDCl_3$ |
|---|---|
| 109 | 3,7 m 8H; 4,25 m 4H; 5,55 s 2H; 7,85 s 1H; 8,50 s 1H |
| 110 | 3,8 m 4H; 4,20 m 4H; 5,45 s 2H; 7,75 s 1H; 8,40 s 1H |
| 111 | 3,75 m 4H; 4,05 m 2H; 4,35 m 2H; 5,53 s 2H; 7,83 s 1H; 8,50 s 1H |
| 178 | 4,65 "t" 2H; 4,85 "t" 2H; 5,30 s 2H; 7,80 s 1H; 8,10 s 1H |
| 205 | 4,25 "t" 2H; 4,55 "t" 2H; 5,32 s 2H; 7,90 s 1H; 8,28 s 1H. |

s: Singulett
"t": Pseudotriplett
m: Multiplett

Die neuen Verbindungen können praktisch alle Entwicklungsstadien einer Pflanze verschiedenartig beeinflussen und werden deshalb als Wachstumsregulatoren oder als Herbizide eingesetzt.

Anwendungsbeispiele

Die herbizide Wirkung von Verbindungen der Formel I wird durch Gewächshausversuche gezeigt:

Als Kulturgefäße dienen Plastikblumentöpfe mit 300 $cm^3$ Inhalt und lehmigem Sand mit etwa 1,5 % Humus als Substrat.

Die Samen der Testpflanzen werden nach Arten getrennt flach eingesät. Unmittelbar danach werden die Wirkstoffe bei Vorauflaufbehandlung auf die Erdoberfläche aufgebracht. Sie werden hierzu in Wasser als Verteilungsmittel suspendiert oder emulgiert und mittels fein verteilender Düsen gespritzt. Die Aufwandmengen betragen dabei 3,0 und 1,0 kg Wirkstoff/ha. Nach dem Aufbringen der Mittel werden die Gefäße leicht beregnet, um Keimung und Wachstum in Gang zu bringen. Danach werden die Gefäße mit durchsichtigen Plastikhauben abgedeckt bis die Pflanzen angewachsen sind. Diese Abdeckung bewirkt ein gleichmäßiges Keimen der Testpflanzen, sofern dies nicht durch die Wirkstoffe beeinträchtigt wird.

Für die Nachauflaufbehandlung werden die Testpflanzen je nach Wuchsform erst bis zu einer Wuchshöhe von 3 bis 15 cm angezogen und danach behandelt. Die für die Nachauflaufanwendung eingesetzten Soja- und Reispflanzen werden in einem mit Torfmull (peat) angereicherten Substrat angezogen, um ein günstigeres Wachstum zu gewährleisten. Zur Nachauflaufbehandlung werden entweder direkt gesäte und in den gleichen Gefäßen aufgewachsene Pflanzen ausgewählt, oder sie werden erst als Keimpflanzen getrennt angezogen und einige Tage vor der Behandlung in die Versuchsgefäße verpflanzt. Eine Abdeckung unterbleibt bei der Nachauflaufbehandlung. Die Aufwandmenge beträgt beispielsweise 1,0 kg Wirkstoff/ha.

Die Versuchsgefäße werden im Gewächshaus aufgestellt, wobei für wärmeliebende Arten wärmere Bereiche (20 bis 30° C) und für solche gemäßigter Klimate 15 bis 25° C bevorzug werden. Die Versuchsperiode erstreckt sich über 2 bis 4 Wochen. Während dieser Zeit werden die Pflanzen gepflegt, und ihre Reaktion auf die einzelnen Behandlungen wird ausgewertet. Bewertet wird nach einer Skale von 0 bis 100. Dabei bedeutet 0 keine Schädigung oder normaler Auflauf und 100 kein Aufgang der Pflanzen bzw. völlige Zerstörung zumidnest der oberirdischen Teile.

Die in den Gwächshausversuchen verwendeten Pflanzen setzen sich aus folgenden Arten zusammen:

| Lateinischer Name | Deutscher Name |
|---|---|
| Amaranthus spp. | Fuchsschwanzarten |
| Centaurea cyanus | Kornblume |
| Chenopodium album | Weißer Gänsefuß |
| Echinochloa crus-galli | Hühnerhirse |
| Galeopsis spp. | Hohlzahn (hempnettle) |
| Galium aparine | Klettenlabkraut |
| Glycine max | Sojabohnen |
| Ipomoea spp. | Prunkwindearten |
| Lamium amplexicaule | Stengelumf. Taubnesel (henbit) |
| Lolium multiflorum | ital. Raygras |
| Lycopersicon lycopersicum | Tomate |
| Mercurialis annua | Einjähriges Bingelkraut |
| Stellaria media | Vogelsternmiere (chickweed) |
| Triticum aestivum | Weizen |
| Viola spp. | Stiefmütterchen |
| Zea mays | Mais |

Bei einer Aufwandmenge von 3,0 kg Wirkstoff/ha entfalten die Verbindungen Nr. 153, 166 und 192 eine beachtliche herbizide Wirkung gegen breitblättrige und grasartige Pflanzen bei Nachauflaufanwendung.

Geeignet zur selektiven Bekämpfung unerwünschter Pflanzen in Kulturen bei einer Aufwandmenge von 0,5 kg Wirkstoff/ha und Nachauflaufanwendung sind die Verbindung Nr. 166 oder mit 1,0 kg Wirkstoff/ha die Verbindungen Nr. 150 und 167. Mit 2,0 kg Wirkstoff/ha bekämpfen die Wirkstoffe Nr. 166 und 204 breitblättrige Unkräuter.

Tabelle 3 (neu) - Selektive Bekämpfung breitblättriger unerwünschter Pflanzen in Weizen bei Nachauflaufanwendung im Gewächshaus

| Wirkstoff Nr. | kg/ha a.S. | Testpflanzen und Schädigung % Triticum* aestivum | Galeopsis spp. | Lamium ampl. | Mercurialis annua | Stellaria media |
|---|---|---|---|---|---|---|
| 166 | 2,0 | 0 | 100 | 100 | 100 | 100 |
| 204 | 2,0 | 0 | 100 | 100 | 98 | 100 |

* Sorte "Vuka"

## Ansprüche

1. Verwendung der Azolylacetophenonoximether der Formel (I)

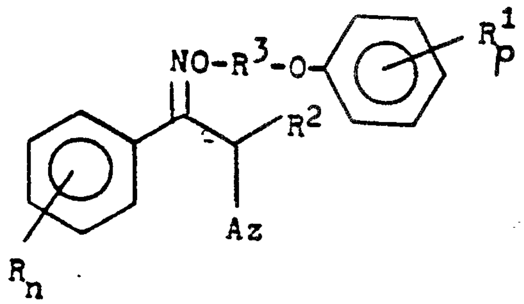

(I),

in denen die Substituenten und die Indices folgende Bedeutung haben:
R und $R^1$ unabhängig voneinander Fluor, Chlor, Brom, $C_1$-$C_5$-Alkyl, Methoxy, Ethoxy, Methylthio, Ethylthio, $C_1$-$C_3$-Halogenalkyl mit bis zu 5 Fluor oder Chlor, Nitro, Cyano, oder ein-, zwei- oder dreifach durch halogen, Cyano, Nitro, oder $C_1$-$C_2$-Halogenalkyl substituiertes Phenyl/Phenoxy;
$R^2$ = Wasserstoff;
$R^3$ = $-(CH_2)_m-$, $-(CH_2CH_2O)_u-CH_2CH_2-$, $-CH_2-CH=CH-CH_2-$, $-CH_2-C\equiv C-CH_2-$,
n, p, u = 1, 2, 3;
m = 2 bis 8;
Az = 1-(1,2,4-Triazolyl), 1-Pyrazolyl,
oder deren Salze zur Bekämpfung unerwünschten Pflanzenwuchses.

2. Verwendung der Verbindungen I, gekennzeichnet durch eine Bedeutung von R, $R^1$, $R^2$, $R^3$, n und p wie in Anspruch 1 angegeben, mit der Maßgabe, daß $R^3$ = $-(CH_2)_m$ - ist und m die Zahlen 2, 3 oder 4 bedeutet, zur Bekämpfung unerwünschten Pflanzenwuchses.

## Claims

1. The use of an azolylacetophenone oxime ether of the formula (I)

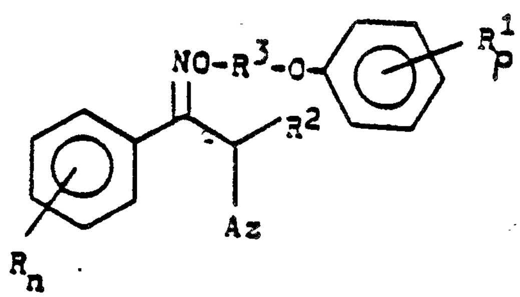

(I)

where R and $R^1$, independently of one another, are each fluorine, chlorine, bromine, $C_1$-$C_5$-alkyl, methoxy, ethoxy, methylthio, ethylthio, $C_1$-$C_3$-haloalkyl of no more than 5 fluorine or chlorine atoms, nitro, cyano, or phenyl or phenoxy both of which are mono-, di- or trisubstituted by halogen, cyano, nitro or $C_1$-$C_2$-haloalkyl; $R^2$ is hydrogen; $R^3$ is $-(CH_2)_m-$, $-(CH_2CH_2O)_u-CH_2CH_2-$, $-CH_2-CH=CH-CH_2-$ or $-CH_2-C\equiv C-CH_2-$; n, p and u are each 1, 2 or 3; m is from 2 to 8; and Az is 1,2,4-triazol-1-yl or pyrazol-1-yl, or the salts thereof for controlling undesirable plant growth.

2. The use of the compound I, wherein R, $R^1$, $R^2$, $R^3$, n and p have the meanings defined in claim 1, with the proviso that $R^3$ is $-(CH_2)_m-$ and m is 2, 3 or 4, for controlling undesirable plant growth.

## Revendications

1. Utilisation des azolylacétophénonoximéthers de la formule (I)

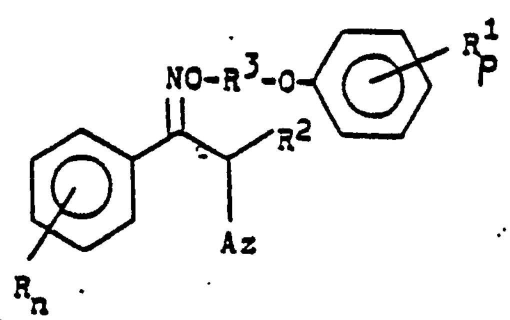

(I),

dans laquelle les substituants et les indices ont les significations suivantes :

R et $R^1$ indépendamment l'un de l'autre, fluor, brome, alkyle en $C_1$-$C_5$, méthoxy, éthoxy, méthylthio, éthylthio, halogénalkyle en $C_1$-$C_3$ avec jusqu'à 5 fluor ou chlore, nitro, cyano ou phényle/phénoxy substitué une, deux ou trois fois par halogène, cyano, nitro, ou halogénalkyle en $C_1$-$C_2$.

$R^2$ = hydrogène

$R^3$ = $-(CH_2)_m-$, $-(CH_2CH_2O)_u-CH_2CH_2-$, $-CH_2-CH=CH-CH_2-$, $-CH_2-C\equiv C-CH_2-$,

n, p, u = 1, 2, 3;

m = 2 à 8;

Az = 1-(1,2,4-triazolyle), 1-pyrazolyle,

ou leurs sels pour lutter contre une croissance indésirable des plantes.

**2.** Utilisation des composés I, pour lutter contre une croissance indésirable des plantes, caractérisé par une signification de R, R', $R^2$, $R^3$, n et p telle que donnée dans la revendication 1, avec la condition que $R^3$ est $-(CH_2)_m-$ et m représente le nombre 2, 3 ou 4.